# EUROPEAN PATENT SPECIFICATION

(11) **EP 3 655 046 B1**
(45) Date of publication and mention of the grant of the patent: **27.01.2021**
(21) Application number: 18756504.9
(22) Date of filing: 18.07.2018
(51) Int. Cl.: A61L 2/26, B66F 7/06, B65G 69/24, B65G 13/00

(54) **APPARATUS AND METHOD TO AUTOMATICALLY INTRODUCE AND REMOVE A SUPPORT FOR CARRYING MEDICAL OBJECTS INTO AND FROM A LARGE-CAPACITY WASHING CHAMBER**
VORRICHTUNG UND VERFAHREN ZUR AUTOMATISCHEN EINFÜHRUNG UND ENTFERNUNG EINES TRÄGERS ZUR AUFNAHME MEDIZINISCHER GEGENSTÄNDE IN UND AUS EINER GROSSRAUMWASCHKAMMER
APPAREIL ET PROCÉDÉ POUR INTRODUIRE ET RETIRER AUTOMATIQUEMENT UN SUPPORT DESTINÉ À TRANSPORTER DES OBJETS MÉDICAUX DANS ET À PARTIR D'UNE CHAMBRE DE LAVAGE DE GRANDE CAPACITÉ

(30) Priority: 18.07.2017 IT 201700081588
(43) Date of publication of application: 27.05.2020
(73) Proprietor: Steelco S.p.A., 31039 Riese Pio X (IT)
(72) Inventor: CAPOVILLA, Ivone, 31037 Loria (IT)
(74) Representative: Petraz, Gilberto Luigi
(86) International application number: PCT/IT2018/050132
(87) International publication number: WO 2019/016841

(56) References cited:
- US-A- 5 829 948
- US-A1- 2004 013 501

## Description

### FIELD OF THE INVENTION

The field of application of the present invention is the washing sector, understood as actual washing proper, disinfection, sterilization, sanitization and any other known operation, or which will be developed hereafter, suitable to return any medical object whatsoever, such as for example instruments, devices, products, containers of drugs or medicines, also liquids, or other, to optimal hygienic conditions for its re-use. More particularly, the present invention is one of the apparatuses and methods to automatically introduce and remove a support for carrying medical objects into and from a large-capacity washing chamber, that is, of at least one or more cubic meters.

### BACKGROUND OF THE INVENTION

In the medical sector, and in particular in that of the washing treatment, understood as above, of the many medical objects which must be treated to be returned to optimal hygienic conditions for their reuse, various apparatuses are known, among which there are those which Applicant has developed, produced and marketed for over a decade and which have also been disclosed through patent publications, such as for example the international patent application published under the number WO-A-2013/068822 and the European patent application EP-A- 3072531.

Among known apparatuses, a particular sector is occupied by those having at least one large-capacity washing chamber, that is, able to accommodate inside itself large-size supports or sliders, for example with a parallelepiped shape with sizes ranging from one to three meters, and even more. One of these known washing chambers marketed by the Applicant is, for example, that marketed under the trade name "Steelco LC80".

The technical problem that the present invention has faced and solved is that of the automatic and safe movement of a support for carrying objects, having a considerable weight, even more than 10,000 N, with respect to a large-capacity washing chamber, in particular from a loading position, outside the washing chamber, to inside the latter, and vice versa.

An apparatus for loading and moving objects, which however has bulky sizes and limited capacity for movement, is described for example in document WO-A-90/15754. On the other side, loading/unloading sliding devices of general use and comprising a lifting platform, are known for example from the patent publication US5829948.

One purpose of the present invention is therefore to provide an apparatus and to perfect the corresponding method to automatically introduce and remove a support for carrying medical objects into and from a large-capacity washing chamber, which are simple and reliable and which on the one hand allow the operator to load the support with the medical objects to be treated, safely and without making excessive efforts, and to remove them from the same support when the treatment has been completed, remaining outside the washing chamber, and on the other hand automatically carry out all the operations of introducing the support into the washing chamber, when loading is complete, and the subsequent removal of the support from the same washing chamber, without the operator ever having to enter the washing chamber.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, an apparatus according to the present invention to automatically introduce and remove a support for carrying medical objects into and from a large-capacity washing chamber, comprises transport means configured at least to take the support from a loading position, in which the support is distant from the washing chamber, to an introduction position, in which the support is in correspondence with the washing chamber.

In accordance with the present invention, the transport means comprise a slider, positioned on a first support plane where the washing chamber lies and translatable in a first sliding direction with respect to the washing chamber by means of first command means; and a platform mounted on the slider and mobile in a second raising and lowering direction perpendicular to the first direction; the platform is mobile between a lowered position, resting on the slider, and a loading position which is raised with respect to the slider, and vice versa, and commanded by second command means; feed means are mounted on the platform, configured to selectively translate the support in a third introduction and removal direction, from a first position in which the support is completely outside the washing chamber, to a second position in which the support is completely inside the washing chamber; the third direction to introduce and remove the support is perpendicular both to the first sliding direction of the slider and also to the second raising and lowering direction of the platform.

The second command means can comprise a fluid-dynamic jack, connected to a mechanism with crossed levers, pivoted with respect to each other, and connected in the lower part to the slider and in the upper part to the platform.

In accordance with another characteristic aspect of the invention, the feed means comprise a plurality of drive wheels mounted rotatable on the platform and which together define a second temporary support plane for the support, parallel to the first support plane.

The drive wheels can be selectively made to rotate, in both directions, by third command means, which can comprise at least a first electric motor connected to the drive wheels.

In accordance with another characteristic aspect of the invention, the feed means also comprise other command means configured both to thrust the support in the last part of its travel inside the washing chamber, and also to begin to remove the support from the washing chamber.

The fourth command means comprises a screw, mounted rotatable in both directions of rotation, around a transverse axis parallel to the third direction, and connected coaxially to a second electric motor, with this screw a corresponding nut can be engaged, integral with the lower and proximal part of an arm, on a distal end of which a fork is present, configured to cooperate with the support.

The fork can be mounted on a shaft rotatably supported by the arm, and the fork can be mobile between an inactive position, in which it is lowered and does not interfere with the support, and a working position, in which it is raised and in cooperation with the support, commanded by fifth command means.

In accordance with another characteristic aspect of the invention, a method to automatically introduce and remove a support that carries medical objects into and from a large-capacity washing chamber, by means of an apparatus as claimed herein, comprises at least a loading step, in which the support is loaded on transport means, in a loading position distant from the washing chamber, and a translation step in which the transport means, comprising a slider commanded by first command means and a platform, take the support from the loading position to an introduction position, in which the support is in correspondence with the washing chamber, in a first sliding direction of the slider parallel to a first support plane where the washing chamber lies. Moreover, the loading step provides to raise the support, commanded by second command means, in a second raising and lowering direction of the platform perpendicular to the first direction, from a lowered position in which the support is on the level of the washing chamber, to a raised position, to facilitate the loading of the medical objects into the support. The method also comprises an introduction step, in which the support is moved by other command means from the outside to the inside of the washing chamber in a third introduction and removal direction of the support which is perpendicular both to the first direction and also to the second direction.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a view from above of an apparatus to automatically introduce and remove a support for carrying medical objects into and from a large capacity washing chamber according to the present invention, shown in a loading position of the support;
- fig. 2 is a lateral view of the apparatus in fig. 1;
- fig. 3 is a front view of the apparatus in fig. 1 shown in a translation position of the slider;
- fig. 4 is an enlarged detail of fig. 3;
- fig. 5 is a view from above of the apparatus in fig. 1, shown in a position of introduction of the support into the washing chamber;
- fig. 6 is a lateral view of the apparatus in fig. 3;
- fig. 7 is a view from above of the apparatus in fig. 1, shown in a position in which the support is completely introduced into the washing chamber;
- fig. 8 is a lateral view of the apparatus in fig. 7;
- fig. 9 is an enlarged and partly sectioned detail of fig. 6;
- fig. 10 is an enlarged and partly sectioned detail of fig. 8.

We must clarify that in the present description and in the claims the terms vertical, horizontal, top, left and below, with their declinations, have the sole function of better illustrating the present invention with reference to the drawings and must not be used in any way to limit the scope of the invention itself, or the field of protection defined by the claims. For example, by the term horizontal we wish to indicate a plane that can be either parallel to the line of the horizon or inclined, even by several degrees, for example up to 10°.

### DETAILED DESCRIPTION OF SOME EMBODIMENTS

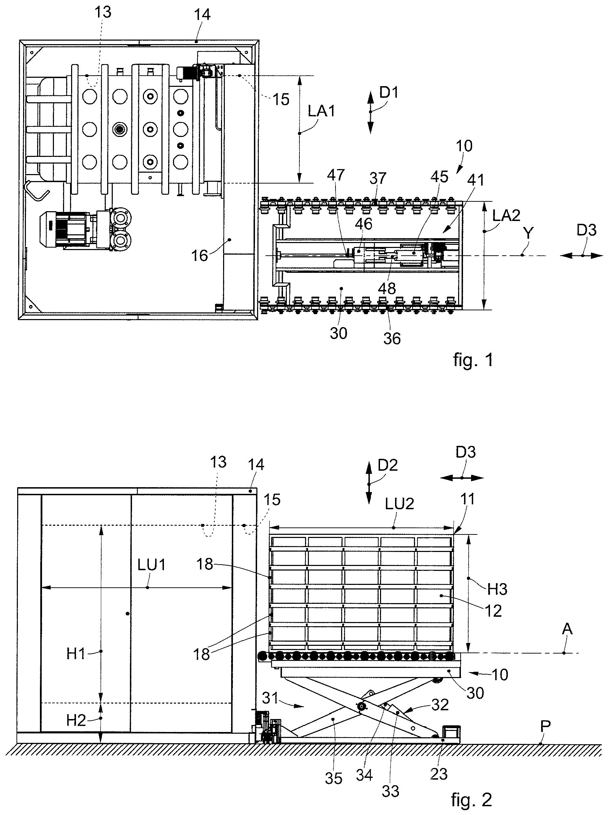

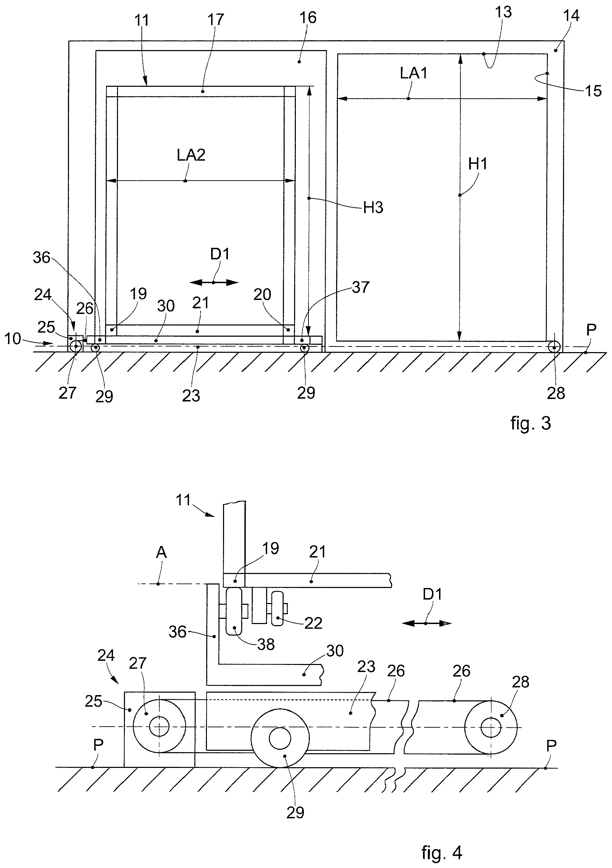

With reference to figs. 1 and 2, an apparatus 10 according to the present invention is configured to automatically introduce and remove a support 11 (figs. 2 and 3) suitable to support a large quantity of medical objects, such as for example surgical instruments, flasks of physiological substances, or other, generally indicated by the reference numeral 12, into and from a large-capacity washing chamber 13, for example about 4 m³, of a washing machine 14 of a known type.

The washing chamber 13 (figs. 1, 2 and 3) has, for example, a substantially parallelepiped shape, with a width LA1 of about 1100 mm, a length LU1 of about 2000 mm and a height H1 of about 1900 mm. The base of the washing chamber 13 is positioned at a height H2, for example about 500 mm from a first support plane P, which can consist of a floor.

In the example provided here, the washing chamber 13 has a single front aperture 15, which can be selectively and hermetically closed by a horizontally sliding door 16.

The support 11 consists, for example, of a structure 17 having a substantially parallelepiped shape and made of metal bars disposed in a grid, which define a plurality of horizontal shelves 18. In particular, the structure 17 comprises in its lower part two lateral bars 19 and 20 (fig. 3) and a front bar 21.

The structure 17, as a whole, has sizes slightly smaller than those of the washing chamber 13, so that the same structure 17 can easily enter and exit the latter. For example, the structure 17 has a width LA2 of about 820 mm, a length LU2 (fig. 2) of about 1650 mm and a height H3 of about 1100 mm, and has quite a big overall weight, for example comprised between about 5,000 N and about 6,000 N, so it would be extremely difficult and unsafe for operators and for the medical objects disposed in it, to move it manually, even by several people at the same time.

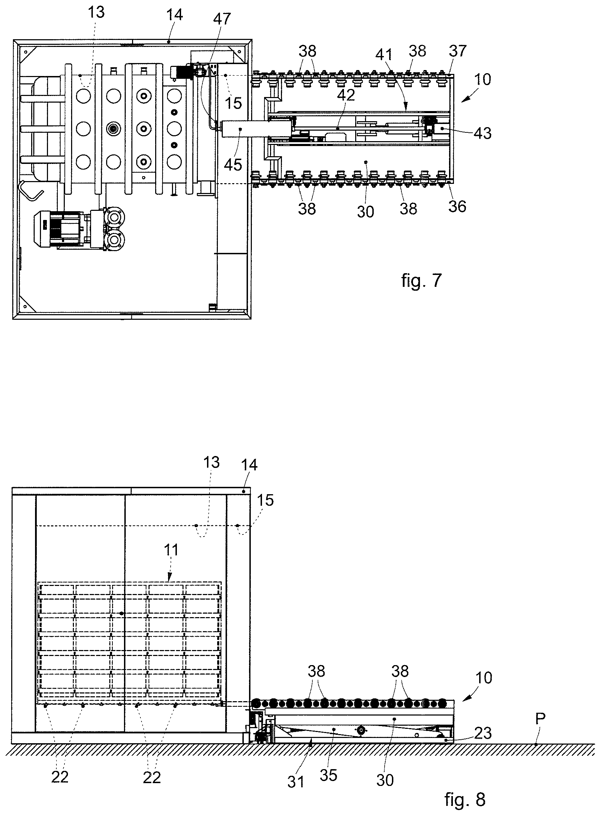

Moreover, the structure 17 is provided, in its lower part, with a plurality of idle wheels 22 (figs. 4 and 8), by means of which it can slide inside the washing chamber 13.

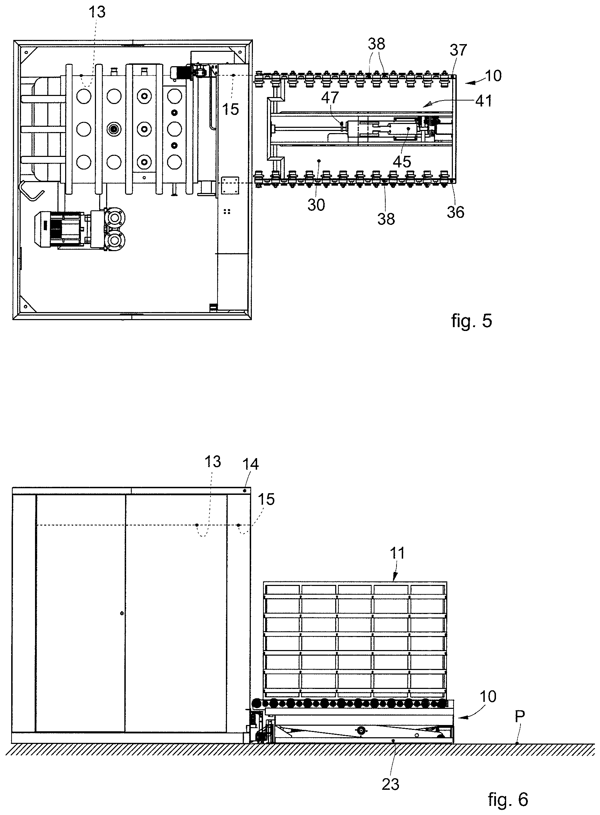

The apparatus 10 comprises a slider 23 configured to translate horizontally in a first direction D1 (figs. 1 and 3) on the support plane P (figs. 2 and 3), between a loading position of the support 11 (figs. 1, 2 and 3), and a position to introduce the support 11 into the washing chamber 13 (figs. 5 to 8), and vice versa, for example by means of first command means 24 (figs. 3 and 4), consisting essentially of an electric motor 25, mounted on the lower front part of the machine 14 and with which a transmission belt 26 is associated, stretched between a drive pulley 27, connected to the electric motor 25, and a driven pulley 28. The slider 23, in its front part, is provided with idle wheels 29 (figs. 3, 4, 9 and 10).

This first direction D1 is therefore a sliding direction of the slider 23 with respect to the washing chamber 13.

The slider 23 therefore slides outside and substantially along a wall of the machine 14 in direction D1 to move toward or away from the front aperture 15 of the washing chamber 13, see for example fig. 1.

A horizontal platform 30 is mounted on the slider 23, and is configured to be selectively raised and lowered in a second vertical direction D2 (fig. 2), keeping itself horizontal, by means of second command means 31, for example consisting of a fluid-dynamic jack 32, for example using oil or compressed air.

The second direction D2 is therefore a raising and lowering direction of the platform 30.

The fluid-dynamic jack 32 has a cylinder 33 integral with the slider 23 and a piston 34 connected to a mechanism with crossed levers 35, pivoted to each other, which is connected in the lower part to the slider 23 and in the upper part to the platform 30 (fig. 2).

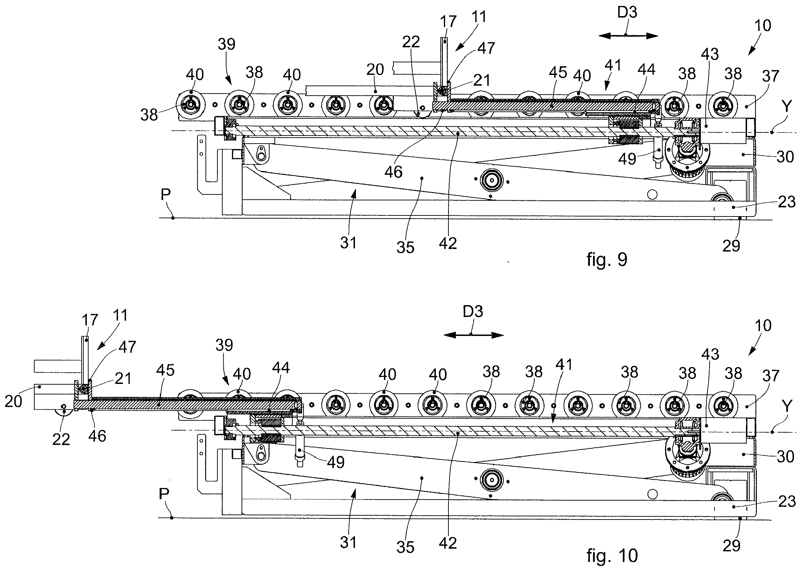

The platform 30 comprises two lateral bars 36 and 37 (figs. 1, 3, 4, 9 and 10) and feed means configured to selectively feed the support 11 in a third direction D3 (figs. 1, 2, 9 and 10), perpendicular both to the first direction D1 and also to the second direction D2, to take it inside the washing chamber 13 and remove it therefrom, as will be described in detail hereafter. The feed means comprise a plurality of drive wheels 38 mounted rotatable on the two lateral bars 36 and 37, and selectively made to rotate in both directions by third command means 39 (figs. 9 and 10), which comprise one or more first electric motors 40 connected to the same drive wheels 38. The drive wheels 38 together define a second temporary support plane A (figs. 2 and 4) for the support 11, parallel to the first support plane P.

The third direction D3 is therefore a direction for introducing and removing the support 11, in particular into and from the washing chamber 13.

The third direction D3 for introducing and removing the support 11 is perpendicular both to the first sliding direction D1 of the slider 23, and also to the second direction D2 for raising and lowering the platform 30.

The feed means also comprise fourth command means 41 (figs. 1, 5, 7, 9 and 10), mounted on the platform 30, in a median position between the two lateral bars 36 and 37, and configured to thrust the support 11 in the last part of its travel inside the washing chamber 13 and to start to remove it therefrom.

The fourth command means 41 comprise a screw 42 (figs. 9 and 10), mounted rotatable, in both directions of rotation, around a transverse axis Y parallel to the third direction D3, and connected coaxially to a second electric motor 43. With the screw 42 a corresponding nut 44 is engaged, integral with the lower and proximal part of an arm 45, on a distal end 46 of which there is a fork 47, configured to cooperate with the front bar 21 of the structure 17. When the arm 45 is in the inactive position (figs. 1 and 5) the fork 47 is positioned around the middle of the platform 30.

In particular, the fork 47 is mounted on a horizontal shaft 48 (fig. 1), supported rotatably by the arm 45, and is mobile between an inactive position, in which it is lowered and does not interfere with the structure 17 (figs. 1 and 5), and a working position, in which it is raised and in cooperation with the front bar 21 of the structure 17 (figs. 7, 9 and 10), commanded by fifth command means 49, consisting, for example, of an electromagnetic actuator, or by a small compressed-air jack.

We must clarify that the five command means 24, 31, 39, 41 and 49 are advantageously controlled by a computerized central unit, of a known type, and not shown in the drawings, and are served by members to detect the position of the various components of the apparatus 10 subject to movement, so that each movement thereof is controlled and is performed automatically and in maximum safety, and can be programmed.

The functioning of the apparatus 10 as described heretofore, which also constitutes the method to automatically introduce and remove the support 11 for medical objects 12 into and from the washing chamber 13, according to the present invention, is as follows.

Initially, the apparatus 10 is in, or is taken to, a loading position (figs. 1, 2 and 3), that is, it is moved to the left with respect to the aperture 15 of the washing chamber 13, with the platform lowered, resting on the slider 23 below. In this initial position, the support 11 is located on the platform 30, so that its lateral bars 19 and 20 (fig. 3), go to rest on the drive wheels 38, which are stationary (fig. 4).

Then follows a step of loading the medical objects 12 in the support 11, which begins with the raising, in the second direction D2, of the platform 30 with respect to the slider 23, which remains stationary in its initial position, to facilitate the operators, who therefore do not need to bend down to dispose the medical objects 12 in the different shelves 18 of the structure 17. The raising of the platform 30 is performed by driving the second command means 31 and the height to which the platform 30 is taken is chosen according to the needs of the operators performing the loading.

When the loading of the support 11 is completed, the second command means 31 are again driven and the platform 30 is returned to its initial position, that is, resting on the slider 23 below.

Then follows a translation step, in which, by activating the first command means 24, the slider 23 is translated, with the already loaded support 11 on board, in the first direction D1 until it is taken into the introduction position, that is, in front of the front aperture 15 of the washing chamber 13, which is open. In fact, in the meantime, the door 16 has been made to slide toward the left part of the machine 14 (fig. 3).

Then follows a step of introducing the support 11, with the medical objects 12 loaded on it, into the washing chamber 13, in the third direction D3. To do this, the third command means 39 are driven, which make all the drive wheels 38 rotate (anti-clockwise in fig. 9), so that the support 11 itself is at least partly taken into the washing chamber 13. In fact, when the front bar 21 of the structure 17 (fig. 9) has reached the position where the fork 47 is, when at rest, some of the drive wheels 38 have lost contact with the lateral bars 19 and 20 of the structure 17 and the thrust of the other drive wheels 38 could be insufficient to complete the travel of the support 11. To overcome this risk, the fifth command means 49 are driven, which take the fork 47 into its working position to engage with the front bar 21 of the structure 17.

The fourth command means 41 are then driven, so that the second electric motor 43 commands the rotation of the screw 42 and consequently the arm 45 takes its distal end 46 and therefore the fork 47, with the front bar 21 of the structure 17 associated with it, as far as the inside of the washing chamber 13 (figs. 7, 8 and 10).

At this point, the whole support 11 with all the medical objects 12 to be treated is completely inside the washing chamber 13.

The fifth command means 49 can then be driven so that the fork 47 returns to its inactive position, and then the fourth command means 41 can be commanded to return the arm 45 back, reversing the direction of rotation of the second electric motor 43 and of the screw 42.

When the arm 45 has reached its initial position (fig. 9), the door 16 can be closed and the machine 14 can begin its treatment cycle on the medical objects 12.

When the treatment is finished, the removal of the support 11 with the medical objects 12 is performed, proceeding in the inverse direction.

After having opened the door 16, the fourth command means 41 are driven, which take the arm 45 with its fork 47 inside the washing chamber 13 and below the support 11.

The fifth command means 49 are then driven to take the fork 47 to engage with the front bar 21 of the structure 17 (figs. 7, 8 and 10).

The fourth command means 41 are then driven, but in the inverse direction, to take the arm 45 toward its initial inactive position, and by means of the fork 47, the arm 45 draws the support 11 with it until the lateral bars 19 and 20 are taken onto the drive wheels 38, which in the meantime have been made to rotate by the third command means 39.

Then the slider 23 is returned by first command means 24 to its initial position (figs. 1, 2 and 3) and there the second command means 31 cause the platform 30 to be raised (fig. 2), to facilitate the removal of the medical objects 12 from the support 11.

Once this last operation has been completed, the support 11 can be reloaded with other medical objects 12 to be treated, or, if necessary, the platform 30 can be lowered by the second command means 31, so that the apparatus 10 returns to its initial starting position.

In the following claims, the sole purpose of the references in brackets is to facilitate reading: they must not be considered as restrictive factors with regard to the field of protection claimed in the specific claims.

## Claims

1. Apparatus (10) configured to introduce and remove a support (11) for carrying medical objects (12) into and from a washing chamber (13) automatically, comprising:
transport means (23, 30) configured at least to take said support (11) from a loading position to an introduction position,
wherein said transport means comprise:
a slider (23), positioned on a support plane (P) and translatable in a first sliding direction (D1) by means of first command means (24); and
a platform (30) mounted on said slider (23) and mobile in a second raising and lowering direction (D2) perpendicular to said first direction (D1), said platform (30) being mobile between a lowered position, resting on said slider (23), and a loading position which is raised with respect to said slider (23) and vice versa, and commanded by second command means (31);
feed means (38, 39, 41, 47) being mounted on said platform (30), configured to feed said support (11) in a third introduction and removal direction (D3), from a first position to a second position;
said third direction (D3) to introduce and remove the support (11) being perpendicular both to said first sliding direction (D1) of the slider (23) and also to said second raising and lowering direction (D2) of the platform (30);
**characterized in that** said feed means also comprise further command means (41), in which said further command means (41) comprise a screw (42), mounted rotatable in both directions of rotation, around a transverse axis (Y) parallel to said third direction (D3), and connected coaxially to a second electric motor (43), and **in that** with said screw (42) a corresponding nut (44) is engaged, integral with the lower and proximal part of an arm (45), on a distal end (46) of which a fork (47) is present, configured to cooperate with said support (11).

2. Apparatus (10) as in claim 1, **characterized in that** said second command means (31) comprise a fluid-dynamic jack (32), connected to a mechanism with crossed levers (35), pivoted with respect to each other, and connected in the lower part to said slider (23) and in the upper part to said platform (30).

3. Apparatus (10) as in claim 1 or 2, **characterized in that** said feed means comprise a plurality of drive wheels (38) mounted rotatable on said platform (30) and which together define a second temporary support plane (A) for said support (11), parallel to said first support plane (P).

4. Apparatus (10) as in claim 3, **characterized in that** said drive wheels (38) are selectively made to rotate, in both directions, by third command means (39) which comprise at least a first electric motor (40) connected to said drive wheels (38).

5. Apparatus (10) as in claim 1, **characterized in that** said fork (47) is mounted on a shaft (48) rotatably supported by said arm (45), and **in that** said fork (47) is mobile between an inactive position, in which it is lowered and does not interfere with said support (11), and a working position, in which it is raised and in cooperation with said support (11), commanded by further command means (49).

6. Method to automatically introduce and remove a support (11) that carries medical objects (12) into and from a washing chamber (13), in particular a large-capacity washing chamber (13), by means of an apparatus according to any one of the preceding claims, comprising a loading step, in which said support (11) is loaded on the transport means (23, 30), in a loading position distant from said washing chamber (13), wherein the method also comprises a translation step in which said transport means (23, 30), comprising the slider (23) commanded by first command means (24) and the platform (30), take said support (11) from said loading position to an introduction position, in which said support (11) is in correspondence with said washing chamber (13), in a first sliding direction (D1) of the slider (23) parallel to the first support plane (P) where said washing chamber lies, wherein said loading step provides to raise said support (11), commanded by second command means (31), in the second raising and lowering direction (D2) of the platform (30) perpendicular to said first direction (D1), from a lowered position in which said support (11) is on the level of said washing chamber (13), to a raised position, to facilitate the loading of said medical objects (12) into said support (11), and wherein said method also comprises an introduction step, in which said support (11) is moved by other command means (39, 41) from the outside to the inside of said washing chamber (13) in the third introduction and removal direction (D3) of the support (11) that is perpendicular both to said first direction (D1) and also to said second direction (D2).

## Patentansprüche

1. Vorrichtung (10), die dazu konfiguriert ist, einen Träger (11) zum Tragen von medizinischen Objekten (12) in eine und aus einer Waschkammer (13) automatisch einzuführen und zu entnehmen, umfassend:
Transportmittel (23, 30), die dazu konfiguriert sind, wenigstens den Träger (11) von einer Ladeposition in eine Einführposition zu bewegen;
wobei die Transportmittel Folgendes umfassen:
einen Schieber (23), der auf einer Auflageebene (P) positioniert und in einer ersten Gleitrichtung (D1) durch ein erstes Befehlsmittel (24) verschiebbar ist; und
eine Plattform (30), die auf dem Schieber (23) angebracht ist und in einer zweiten Hebe- und Senkrichtung (D2) senkrecht zur ersten Richtung (D1) beweglich ist, wobei die Plattform (30) zwischen einer abgesenkten Position, in der sie auf dem Schieber (23) ruht, und einer Ladeposition, in der sie in Bezug auf den Schieber (23) angehoben ist, und umgekehrt beweglich ist und durch das zweite Befehlsmittel (31) gesteuert wird;
Zuführmittel (38, 39, 41, 47), die auf der Plattform (30) angebracht und dazu konfiguriert sind, den Träger (11) in einer dritten Einführ- und Entnahmerichtung (D3) von einer ersten Position in eine zweite Position zuzuführen; wobei die dritte Richtung (D3) zum Einführen und Entnehmen des Trägers (11) sowohl zur ersten Gleitrichtung (D1) des Schiebers (23) als auch zur zweiten Hebe- und Senkrichtung (D2) der Plattform (30) senkrecht ist;
**dadurch gekennzeichnet, dass** die Zuführmittel außerdem ein weiteres Befehlsmittel (41) aufweist, wobei das weitere Befehlsmittel (41) eine Schraube (42) umfasst, die in beide Drehrichtungen um eine zur dritten Richtung (D3) parallele Querachse (Y) drehbar angebracht ist und koaxial mit einem zweiten Elektromotor (43) verbunden ist, **und dass** mit der Schraube (42) eine entsprechende Mutter (44) in Eingriff steht, die fest mit dem unteren und proximalen Teil eines Arms (45) verbunden ist, an dessen distalem Ende (46) sich eine Gabel (47) befindet, die dazu konfiguriert ist, mit dem Träger (11) zusammenzuwirken.

2. Vorrichtung (10) nach Anspruch 1, **dadurch gekennzeichnet, dass** das zweite Befehlsmittel (31) einen fluiddynamischen Zylinder (32) umfasst, der mit einem Mechanismus mit gekreuzten Hebeln (35) verbunden ist, die in Bezug zueinander schwenkbar sind und im unteren Abschnitt mit dem Schieber (23) und im oberen Abschnitt mit der Plattform (30) verbunden sind.

3. Vorrichtung (10) nach Anspruch 1 oder 2, **dadurch gekennzeichnet, dass** die Zuführmittel eine Vielzahl von Antriebsrädern (38) umfassen, die drehbar auf der Plattform (30) angebracht sind und die zusammen eine zweite temporäre Auflageebene (A) für den Träger (11) parallel zu der ersten Auflageebene (P) definieren.

4. Vorrichtung (10) nach Anspruch 3, **dadurch gekennzeichnet, dass** die Antriebsräder (38) durch dritte Befehlsmittel (39), die mindestens einen ersten Elektromotor (40) umfassen, der mit den Antriebsrädern (38) verbunden ist, in beide Richtungen selektiv in Drehung versetzt werden.

5. Vorrichtung (10) nach Anspruch 1, **dadurch gekennzeichnet, dass** die Gabel (47) auf einer Welle (48) angeordnet ist, die drehbar von dem Arm (45) getragen wird, **und dass** die Gabel (47), gesteuert durch weitere Befehlsmittel, zwischen einer inaktiven Position, in der sie abgesenkt ist und nicht mit dem Träger (11) kollidiert, und einer Arbeitsposition beweglich ist, in der sie angehoben ist und mit dem Träger (11) zusammenwirkt.

6. Verfahren zum automatischen Einführen und Entnehmen eines Trägers (11), der medizinische Objekte (12) trägt, in eine und aus einer Waschkammer (13), insbesondere einer Waschkammer (13) mit großem Fassungsvermögen, mittels einer Vorrichtung gemäß einem der vorhergehenden Ansprüche, umfassend einen Ladeschritt, bei dem der Träger (11) auf die Transportmittel (23, 30) in einer Ladeposition geladen wird, die von der Waschkammer (13) beabstandet ist, wobei das Verfahren außerdem einen Verschiebeschritt umfasst, in dem die Transportmittel (23, 30), die den Schieber (23), der von dem ersten Befehlsmittel (24) gesteuert wird, und die Plattform (30) umfassen, den Träger (11) von der Ladeposition in eine Einführposition bringen, in welcher der Träger (11) mit der Waschkammer (13) in einer ersten Gleitrichtung (D1) des Schiebers (23) parallel zur ersten Auflageebene (P) korrespondiert, in der die Waschkammer liegt, wobei der Ladeschritt ein Anheben des Trägers (11), das durch ein zweites Befehlsmittel (31) gesteuert wird, in der zweiten Hebe- und Senkrichtung (D2) der Plattform (30) senkrecht zur ersten Richtung (D1) von einer abgesenkten Position, in der sich der Träger (11) auf der Höhe der Waschkammer (13) befindet, in eine angehobene Position vorsieht, um das Laden der medizinischen Objekte (12) in den Träger (11) zu erleichtern, und wobei das Verfahren auch einen Einführschritt umfasst, in dem der Träger (11) durch andere Befehlsmittel (39, 41) von der Außenseite zur Innenseite der Waschkammer (13) in der dritten Einführ- und Entnahmerichtung (D3) des Trägers (11) bewegt wird, die senkrecht sowohl zur ersten Richtung (D1) als auch zur zweiten Richtung (D2) steht.

## Revendications

1. Appareil (10) configuré pour introduire et retirer un support (11) destiné à transporter automatiquement des objets médicaux (12) dans et depuis une chambre de lavage (13), comprenant :
des moyens de transport (23, 30) configurés au moins pour amener ledit support (11) d'une position de chargement à une position d'introduction,
dans lequel lesdits moyens de transport comprennent :
un coulisseau (23), positionné sur un plan de support (P) et pouvant être déplacé en translation dans une première direction de coulissement (D1) par l'intermédiaire de premiers moyens de commande (24) ; et
une plate-forme (30) montée sur ledit coulisseau (23) et mobile dans une deuxième direction d'élévation et d'abaissement (D2) perpendiculaire à ladite première direction (D1), ladite plate-forme (30) étant mobile entre une position abaissée, reposant sur ledit coulisseau (23), et une position de chargement qui est élevée par rapport audit coulisseau (23) et vice versa, et commandée par des seconds moyens de commande (31) ;
des moyens d'alimentation (38, 39, 41, 47) étant montés sur ladite plate-forme (30), configurés pour alimenter ledit support (11) dans une troisième direction d'introduction et de retrait (D3), d'une première position à une seconde position ;
ladite troisième direction (D3) pour introduire et retirer le support (11) étant perpendiculaire à la fois à ladite première direction de coulissement (D1) du coulisseau (23) et également à ladite deuxième direction d'élévation et d'abaissement (D2) de la plate-forme (30) ;
**caractérisé en ce que** lesdits moyens d'alimentation comprennent également des moyens de commande supplémentaires (41), dans lesquels lesdits moyens de commande supplémentaires (41) comprennent une vis (42), montée rotative dans les deux directions de rotation, autour d'un axe transversal (Y) parallèle à ladite troisième direction (D3), et connectée coaxialement à un second moteur électrique (43), et **en ce qu'**un écrou correspondant (44) est mis en prise avec ladite vis (42), d'un seul tenant avec la partie inférieure et proximale d'un bras (45), sur une extrémité distale (46) dont une fourche (47) est présente, configurée pour coopérer avec ledit support (11).

2. Appareil (10) selon la revendication 1, **caractérisé en ce que** lesdits seconds moyens de commande (31) comprennent un vérin fluidodynamique (32), relié à un mécanisme à leviers croisés (35), pivotant l'un par rapport à l'autre, et relié dans la partie inférieure audit coulisseau (23) et dans la partie supérieure à ladite plateforme (30).

3. Appareil (10) selon la revendication 1 ou 2, **caractérisé en ce que** lesdits moyens d'alimentation comprennent une pluralité de roues d'entraînement (38) montées rotatives sur ladite plate-forme (30) et qui définissent ensemble un deuxième plan de support temporaire (A) pour ledit support (11), parallèle audit premier plan de support (P).

4. Appareil (10) selon la revendication 3, **caractérisé en ce que** lesdites roues d'entraînement (38) sont mises en rotation sélectivement, dans les deux directions, par des troisièmes moyens de commande (39) qui comprennent au moins un premier moteur électrique (40) relié à lesdites roues motrices (38).

5. Appareil (10) selon la revendication 1, **caractérisé en ce que** ladite fourche (47) est montée sur un arbre (48) supporté en rotation par ledit bras (45), et **en ce que** ladite fourche (47) est mobile entre une position inactive, dans laquelle elle est abaissée et n'interfère pas avec ledit support (11), et une position de travail, dans laquelle elle est élevée et en coopération avec ledit support (11), commandée par des moyens de commande supplémentaires (49).

6. Procédé pour introduire et retirer automatiquement un support (11) qui transporte des objets médicaux (12) dans et depuis une chambre de lavage (13), en particulier une chambre de lavage de grande capacité (13), au moyen d'un appareil selon l'une quelconque l'une des revendications précédentes, comprenant une étape de chargement, dans laquelle ledit support (11) est chargé sur les moyens de transport (23, 30), dans une position de chargement éloignée de ladite chambre de lavage (13), dans lequel le procédé comprend également une étape de déplacement translation dans laquelle lesdits moyens de transport (23, 30), comprenant le curseur (23) commandé par les premiers moyens de commande (24) et la plateforme (30), amènent ledit support (11) de ladite position de chargement à une position d'introduction, dans laquelle ledit support (11) est en correspondance avec ladite chambre de lavage (13), dans une première direction de coulissement (D1) du coulisseau (23) parallèle au premier plan de support (P) où se trouve ladite chambre de lavage, dans lequel ladite étape de chargement prévoit d'élever ledit support (11), commandé par des deuxièmes moyens de commande (31), dans le deuxième direction d'élévation et d'abaissement (D2) de la plate-forme (30) perpendiculaire à ladite première direction (D1), d'une position abaissée dans laquelle ledit support (11) est au niveau de ladite chambre de lavage (13), à une position élevée, pour faciliter le chargement desdits objets médicaux (12) dans ledit support (11), et dans lequel ledit procédé comprend également une étape d'introduction, dans laquelle ledit support (11) est déplacé par des moyens de commande supplémentaires (39, 41) de l'extérieur vers l'intérieur de ladite chambre de lavage (13) dans la troisième direction d'introduction et de retrait (D3) du support (11) qui est perpendiculaire à la fois à ladite première direction (D1) et également à ladite deuxième direction (D2).
